# EUROPEAN PATENT APPLICATION

(11) **EP 4 339 293 A1**
(43) Date of publication of application: **20.03.2024**
(21) Application number: 22195543.8
(22) Date of filing: 14.09.2022
(51) Int. Cl.: C12P 19/02, C12P 19/04, C12P 19/14

(54) **METHOD FOR ENZYMATIC MODIFICATION OF STARCH**

(71) Applicant: Südstärke GmbH, 86529 Schrobenhausen (DE)
(72) Inventor: Lang Halter, Evi, 86529 Schrobenhausen (DE)
(74) Representative: Hoefer & Partner Patentanwälte mbB

(57) **Abstract**

The present invention is directed to a method for enzymatic modification of starch, comprising the following steps: (a) preparing a suspension comprising starch and water; (b) applying the suspension to a drum of a drum drying device, wherein the suspension is not heated above 70 °C, in particular not above 60 °C before applying to the drum drying device; (c) adding at least one enzyme to the suspension before or after the suspension is applied to the drum drying device; (d) heating the suspension containing the at least one enzyme on the drum drying device to temperatures above 70 °C, in particular above 80 °C; (e) obtaining the enzymatically modified starch from the drum drying device. A further aspect of the invention is directed to the use of a drum drying device for the gelatinization of a suspension comprising native starch, water and at least one enzyme, wherein the suspension was not heated to a temperature above 70 °C, preferably not above 60 °C, in particular not above 50 °C before applying to the drum drying device.

## Description

### Field of the Invention

The present invention concerns a method for enzymatic modification of starch using a drum drying device. In general, a suspension comprising starch and water is prepared which is applied to a drum of a drum drying device in a subsequent step. The suspension, to which at least one enzyme is added before or after application to the drum of a drum drying device, is not heated above 60°C before application to the drum.

### Technical Background

A number of methods for enzymatic modification of starch are known in the art. Native starches are modified in such processes in order to overcome the shortcomings, including retrogradation, syneresis and low water-holding potential, which limit their industrial applications.

In the art, various processes are known in which a suspension comprising native starch is treated at elevated temperature, typically above 70°C with at least one starch-modifying enzyme. At this elevated temperature, the native starch grains are affected and the gelatinized starch is modified by the action of the at least enzyme regarding its structure and properties.

The enzyme-modified starch product is then further processed.

For example, CN105211790A discloses a preparation method for enzyme-modified lotus root starch. The preparation method is performed according to the following steps: (1) Preparation of raw materials: collecting lotus root starch powder, removing impurities, and smashing for use; (2) Enzymatic hydrolysis of the powder: the raw material of the step (1) is placed in a container, and the water and the raw material are added to the container at a ratio of 1 to 20:1 by weight, and the mixture is stirred at room temperature to 100 °C; 1000 to 100000 U/kg adding alpha amylase in proportion to the raw material are added and maintained at a temperature of 80 - 110 °C for 0.1-200 min; (3) Drying: drying the powder hydrolysate obtained in the step (2) by drum drying or spray drying to obtain an off-white powder; (4) Granulation: the powder obtained in the step (3) is granulated and dried in a fluidized bed to obtain a granulated product, which is a low-temperature pulverized powder product.

There is a need in the art for providing an improved method for the simple and efficient preparation of an enzymatically modified starch, in particular having low viscosity and excellent cold water solubility. In particular, there is a constant need for a simplified process which minimizes both the number and complexity of the process steps, the time needed for the process and the equipment required.

### Summary of the Invention

A first aspect of the present invention concerns a method for enzymatic modification of starch, comprising the following steps:
a) preparing a suspension comprising starch and water,
b) applying the suspension to a drum drying device,
wherein the suspension is not heated above 70 °C, in particular not above 60 °C before applying to the drum drying device,
c) adding at least one enzyme to the suspension before or after the suspension is applied to the drum drying device,
d) heating the suspension containing the at least one enzyme on the drum drying device to temperatures above 70 °C, in particular above 80 °C,
e) obtaining the enzymatically modified starch from the drum drying device.

Other steps may or may not be included.

A further aspect of the present invention concerns the use of a drum drying device for the gelatinization of a suspension comprising native starch, water and at least one enzyme, wherein the suspension was not heated to a temperature above 70 °C, preferably not above 60 °C, in particular not above 50 °C before applying to the drum drying device.

### Detailed Description of the Invention

The inventors have found that the method of the invention as described herein surprisingly allows the production of an advantageous, enzymatically modified starch product by a simple and straightforward process, using standard equipment, in particular a (conventional) drum drying device, avoiding extra process steps such as an incubation step with an enzyme at elevated temperature before application to the drum drying device.

In step (a), a suspension comprising starch and water is prepared. The preparation of such a suspension comprising starch and water is known in the art. The starch is in particular a native starch. Native starch is not chemically or physically modified and contains native starch granules. Waxy starches and high amylose starches, i.e. starches with a very high amylopectin content or a very high amylose content are also considered as suitable for the method according to the invention. The starch may be obtained from any starch-producing plant, and may - without limitation - in particular be potato starch, maize starch, corn starch, starch from tapioca, arrowroot, wheat, rice, sago, mung bean or the like. Potato starch is particularly preferred. Preferably, unwanted impurities originating from the respective plant source material are removed.

Also, as a native starch, the starch has typically not been subjected to any enzymatic modification, or a heat treatment (see also below), which destroys the starch grains of the native starch.

The native starch is suspended in water in any conventional way known in the art. Apart from starch and water, the suspension may or may not contain additional ingredients, such as buffers/ pH-adjusting agents, salts or enzyme activators. In a preferred embodiment of the present invention, the suspension of step a) essentially or completely consists of native starch and water.

Herein, the suspension comprising starch and water is also called starch-containing suspension in a synonymous way. Once this suspension has been heated according to step d) as described herein (above the gelatinization temperature of the starch) it is strictly speaking no longer a suspension, so that also the term starch-containing composition is used hereinafter.

According to a preferred embodiment of the present invention, the starch-containing suspension (or slurry) of step (a) preferably comprises a mixture of starch and water in a weight ratio of 2:1 to 1:2, in particular 0.8:1 to 1.2:1, such as about 1:1. It has been found that in many cases this weight ratio is advantageously high and suitable for applying the suspension to a drum drying device. Typically, the starch-containing suspension has a density of about 22 to 24 Baumé, although other values may be used as appropriate.

As an important feature of the present invention, see step (b) above, before applying to a drum drying device, the (native) starch and the respective suspension containing it is not heated to an elevated temperature at or above the gelatinisation temperature of the starch. Gelatinisation temperatures for native starch of various sources are known in the art. In one embodiment, the gelatinisation temperature can be determined as follows: measurement of viscosity using a Viscograph E device (Brabender GmbH & Co. KG, DE) according to the following settings: 5 wt.% suspension of sample in aqua dest; measuring range 700 cmg; speed 75 rpm; start temperature 20°C; max temperature 95°C, hold 10 min; slope 3°C/min incubation time 25 min. The gelatinization temperature is detected by the rapid increase of viscosity when increasing the temperature; it is indicated on the plot of the temperature gradient versus viscosity by the software of the Brabender Viscograph E. A typical native potato starch may be gelatinised at about 63°C, and max viscosity may be observed at about 73°C.

Preferably, the suspension comprising starch and water according to step (a) (with or without the presence of at least one enzyme) is not heated above about 60°C, more preferably not above about 50°C, in particular not above about 40°C. In one preferred embodiment, the starch-containing suspension is prepared and kept (substantially) at room temperature until it is applied to the drum drying device.

Avoiding heating of the starch-containing suspension to an elevated temperature, in particular above the gelatinization temperature of the starch before the application to the drum drying device ensures that the starch grains are essentially intact and not immediately degraded in the presence of an enzyme (such as alpha-amylase) added to the starch-containing suspension before the application to the drum drying device. This is in clear contrast to the teaching in the prior art wherein a degradation and gelatinization of the starch by the action of enzyme(s) and heat is performed on purpose prior to applying the composition to a drying device. The inventors have now surprisingly found that starting the enzyme degradation and gelatinization of the starch at elevated temperature only after application of a starch-containing composition to the drum drying device, without prior heating, is advantageous in several respects as further set out herein.

Any conventional drum drying device may be used as known in the art. Typically, in drum drying, the heated surface is the envelope of a rotating horizontal metal cylinder. The cylinder is heated by steam condensing inside, typically at a pressure in the range of 200 kPa to 500 kPa, bringing the temperature of the cylinder wall typically to a final temperature of about 120°C to 180°C. The wet material (starch-containing suspension optionally containing the at least one enzyme), is applied to the drum surface as a relatively thin layer. The dried product is removed from the drum, typically with the help of a blade or the like. A typical drum dryer may often have either a single drum with smaller applicator rolls or double drums.

Before or after step (b) applying the suspension comprising starch and water to a drum drying device, at least one enzyme for enzymatic modification of the starch ("starch-modifying enzyme") is added.

Suitable enzymes are known to the skilled person in the art. According to a preferred embodiment of the present invention, the enzyme is selected from the group of starch-modifying enzymes, in particular from one or more of the group of endo-amylases cleaving 1,4-α-D glucosidic bonds, glucoamylases cleaving 1,4 and 1,6-α-D glucosidic linkages, glycosyltransferases, or pullulanases, or mixtures thereof. In a further preferred embodiment, the enzyme(s) comprise(s) of consist(s) of one or more alpha amylases.

Regarding the amount of enzyme(s) it has been found that concentrations of about 0.01% to 3% by weight per weight of (native) starch in the suspension are typically preferred and allow advantageous results. Depending on the type of enzyme, the amount chosen may be adjusted as desired. Typical ranges (units per gram of starch) are the following: Amylases 0.01 to 1000 U (units)/g, preferably 0.05 to 500 U/g; Glucoamylases 0.1 to 5000 U/g, preferably 0.5 to 2500 U/g; Glycosyltransferases: 0.1 to 30000 U/g, preferably 0.5 to 15000 U/g. As an Example, when using the commercial alpha-Amylase BAN^{®} 800MG (Univar Solutions, UK) for the modification of 1 kg native starch as low as about 80 U can be applied.

According to step c), at least one enzyme is added to the suspension comprising starch and water before or after the suspension is applied to the drum drying device. Of course, it is in principle also possible to add water and enzyme at the same time to the (dry) starch in order to obtain the starch-containing suspension.

According to one embodiment of the present invention, the at least one enzyme is added to the starch-containing suspension before applying it to the drum drying device, preferably less than 10 minutes, in particular less than 5 minutes before. In a preferred embodiment of the present invention, the at least one enzyme is added to the starch-containing suspension immediately before or at the time of applying it to the drum drying device. "Immediately before" as used herein shall mean less than 2 minutes, preferably less than 1 minute before. "At the time of' as used herein shall mean that the at least one enzyme is added to the starch-containing suspension concomitantly with applying the suspension on the drum drying device. For example, an enzyme solution/suspension may be sprayed/poured to the drum(s) of a drum drying device together with the starch-containing suspension. According to a preferred embodiment of the invention, the at least one (starch-modifying) enzyme is added at a temperature below the gelatinization temperature of the starch. According to a further preferred embodiment of the invention, the at least one starch-modifying enzyme is added at a temperature of less than 50°C, in particular less than 40°C.

In general, the starch-containing suspension (and the at least one enzyme, as applicable) is added to the drum drying device in any conventional way. For example, it is applied by spraying or pouring onto a rotating drum of the drum drying device. Often, two rotating drums are used and the fluid feed is applied between them.

According to one embodiment, it may also be applied to an applicator roll of the drum drying device.

According to a preferred embodiment, the at least one enzyme is added to the piping (tube) used to forward the starch suspension (slurry) to the drum drying device, in particular an applicator roll thereof.

When the enzyme is added to the starch-containing suspension at the time of (i.e. concomitant with) applying the latter to the drum drying device, it is preferred that it is applied between two rotating rolls or to an applicator roll of the drum drying device as this optimizes homogenous distribution of the at least one enzyme in the starch-containing suspension before it is heated on the heated drum(s) of the drum drying device.

According to a further embodiment of the present invention, the at least one enzyme is added to the starch-containing suspension after applying it to the drum of a drum drying device, preferably less than 5 seconds after applying it to the drum, so that the enzyme is present and can modify the starch once it is heated above the gelatinization temperature on the heated drum of the drum drying device. Again, spraying, sprinkling or pouring the at least one enzyme (in the form of a solution) on the starch-containing composition on the drum may be advantageously used to evenly distribute the enzyme(s).

In an alternative embodiment of the present invention, the drum drying device is replaced by a belt drying device, i.e. the heated surface is not a cylinder surface, but a belt conveyor heated by contact or radiation by hot elements installed on one or both sides.

According to step d), within the present invention it is important that only after application to the drum drying device, the suspension comprising starch and water is heated on the drum drying device, preferably in the presence of at least one enzyme added to the suspension, to temperatures above 70°C, in particular above 80°C.

It was surprisingly found that his allows the suspension to be optimized for application to the drum drying device without having to take into account any continuous and ongoing change of the viscosity or density of the suspension due to the action of the at least one enzyme in the suspension, as is the case if such a suspension is heated to an elevated temperature in the presence of the at least enzyme prior to the application to the drum of a drum drying device.

The invention thus avoids that the optimum constitution of the suspension for applying to the drum drying device is a kind of "moving target" difficult to optimize. It further allows avoiding an extra incubation step and the respective equipment and heating required therefore, and shortens the time needed for the method according to the invention accordingly.

Surprisingly, it was found that the time during which the starch-containing composition is typically present on the drum of a conventional drum drying device is sufficient to allow the gelatinisation of the starch as well as the desired action of the enzyme on the (gelatinised) starch in order to obtain the desired product, in particular a low viscosity and/or partially or completely cold water soluble starch product.

A typical residence time, i.e. time period from applying the suspension comprising starch and water (and enzyme) to the drum drying device and removal of the (gelatinized, dried and enzymatically modified) starch composition from the heated drum may be from about 10 seconds to about 2 minutes, in particular about 10 seconds to about 1 min.

In a preferred embodiment, the time period during which the composition containing starch and enzyme remains on the heated drum of the drum drying device is in the range of 10 seconds to 2 minutes, in particular 10 seconds to 1 minute. The preferred temperature of the heated drum is 130 to 180°C, in particular 140 to 160°C.

According to a preferred embodiment, the starch obtained in step (e) above has a low viscosity of less than 50 mPa*s, preferably less than 30 mPa*s, more preferably less than 20 mPa*s. This viscosity may be determined according to the methodology as described further below.

According to a preferred embodiment, the starch product obtained in step e) has a cold water solubility of 50 to 100%, in particular about 100%.

It is further preferred within the method according to the present invention that the temperature of the starch-containing composition containing the at least one enzyme is raised on the drum drying device to a final temperature of not less than 120°C, preferably not less than 130°C. Moreover, the temperature is typically below 180°C, preferably at most 160°C. A preferred range is from about 140 to about 160°C. It is preferred that the maximum temperature to which the starch-containing composition is heated on the drum drying device ensures the inactivation of the respective at least one enzyme used in the method of the present invention, i.e. that no further enzyme activation step is needed after the drum drying step.

A further aspect of the present invention is directed to the use of a drum drying device for the gelatinisation of a suspension comprising native starch, water and least one enzyme, wherein the suspension was not heated to a temperature above 70°C, preferably not above 60°C, more preferably not above 50°C, in particular not above 40°C before applying to the drum drying device.

As set out above, this use of a drum drying device allows a one-step production of an enzymatically modified starch, optimizing applicability of the suspension to the drum drying device as well as fast and efficient preparation of the desired, enzymatically modified starch product in a dried form.

### Methods:

### Viscosity of starch obtained by the methods of the invention

This can be measured according to a customized Brabender method (Brabender Viscograph Type E, measuring range 250cmg, temperature gradient 25°C to 30°C) by mixing 30g dry starch to 55g ethylene glycol and 400g distilled water. End viscosity was measured at a temperature of 30°C. "Dry starch" shall mean that the material was dried to constant weight at 120°C in an oven (normally achieved after about 8-10 min).

### Determination of gelatinization temperature of starch:

The gelatinisation temperature can be determined as follows: measurement of viscosity using a Viscograph E device (Brabender GmbH & Co. KG, DE) according to the following settings: 5 wt.% suspension of sample in aqua dest; measuring range 700 cmg; speed 75 rpm; start temperature 20°C; max temperature 95°C, hold 10 min; slope 3°C/min incubation time 25 min. The gelatinization temperature is detected by the rapid increase of viscosity when increasing the temperature; it is indicated on the plot of the temperature gradient versus viscosity by the software of the Brabender Viscograph E.

### Cold water solubility of starch:

Determination of cold water solubility of starch is known in the art. According to a preferred embodiment, it can be performed according to the method of Höppler, see e.g. O. Wolff, "Die Stärke" 11, Seiten 273-279, 1950, Kapitel "Dextrin. Herstellung, Untersuchungsmethoden und Gütebestimmungen", Section 3. "Kaltwasserlöslichkeit" on page 276.

The cold water solubility of starch can alternatively be determined by measuring the refractive index of a starch solution. 10g of starch on dry weight basis are dispersed in 90g distilled water. The suspension is filtrated through a folded filter and the obtained solution is measured by means of a refractometer. The determined refractive index is directly correlated to the degree of cold water solubility. "On dry weight basis" shall mean that the material was dried to constant weight at 120°C in an oven (normally achieved after about 8-10 min).

### Examples

In the following, the invention will be further illustrated by the following examples. These examples are only for illustrating the invention without restricting its scope, which is defined by the attached claims.

### Example 1:

A mixture of native potato starch and water, in a 1:1 weight ratio having a density of 23 Bàume was prepared. This suspension was further used in Samples 1A, 1B as follows:

### Sample 1A:

A first sample of the aforementioned starch suspension (Sample 1A) was placed in a container heated to 100°C under stirring. Alpha-amylase (BAN^{®} 800MG, Univar Solutions, UK) in an amount of 80U/kg of starch was added under stirring and the temperature was maintained at 100°C for 0.1 min, before applying the material to the applicator drum of a conventional drum drying device (heated drum: 150°C; residence time on heated drum: 30 sec).

It was observed that the application to the drum of a drum drying device was problematic as the viscosity of the material constantly changed (rapidly decreased) and due to the ongoing enzymatic activity film formation and reliable applicability of the desired film thickness on the drum was strongly impaired. In some repetitions, the film started dripping from the drum surface before sufficient drying. See Table 1 for a summary of the results.

### Sample 1B:

To a second sample of the aforementioned starch suspension (Sample 1B), the same amount of enzyme was added under stirring, however without any heating of the suspension prior to applying it to the drum drying device. Application of the composition comprising starch, enzyme and water to the drum drying device was otherwise performed in the same way and on the same drum drying device (heated drum: 150°C; residence time on heated drum: 30 sec) as described above for Sample 1A.

It was observed that application of the suspension on to the drum of the drum drying device was much more convenient and reliable, due to the lack of (continuous) change of viscosity. Thus, it appeared that the enzyme could not influence the constitution of the slurry at the low temperature and the presence of intact starch grains in the suspension applied to the drum.

At the same time, surprisingly, the residence time of the material on the heated drum of the conventional drum drying device (about 30 sec in the present case for Samples 1A an 1B) was sufficient to perform both starch gelatinisation as well as enzymatic modification of the (gelatinised) starch material on the heated drum, in order to obtain a low-viscosity starch product having 100% cold water solubility. See Table 1 for a summary of the results.

**Table 1:**

| Sample | Heated to above 70°C before application to drum drying device? | Starch gelatinized before application to drum drying device? | Composition easily applicable to drum as steady constant film? |
|---|---|---|---|
| 1A | + | + | - |
| 1B | - | - | + |

According to the invention, gelatinization and viscosity reduction / enzyme reaction occur contemporaneously once the slurry is in contact with the heated surface of the drum. Quick drying is advantageously performed directly on the same drum / device. This provides a true one step procedure.

## Claims

1. A method for enzymatic modification of starch, comprising the following steps:
a) preparing a suspension comprising starch and water,
b) applying the suspension to a drum drying device,
wherein the suspension is not heated above 70 °C, in particular not above 60 °C before applying to the drum drying device,
c) adding at least one enzyme to the suspension before or after the suspension is applied to the drum drying device,
d) heating the suspension containing the at least one enzyme on the drum drying device to temperatures above 70 °C, in particular above 80 °C,
e) obtaining the enzymatically modified starch from the drum drying device.

2. The method according to claim 1, wherein the suspension is not heated above 50 °C, in particular not above 40 °C before applying to the drum drying device.

3. The method of any of the preceding claims, wherein the suspension is not heated above the gelatinization temperature of the starch before applying to the drum drying device.

4. The method of any of the preceding claims, wherein the starch used in step a) of claim 1 is a native starch, preferably from potato.

5. The method of any of the preceding claims, wherein the enzyme is selected from the group of starch-modifying enzymes, preferably from one or more of the group of endo-amylases cleaving 1,4-α-D glucosidic bonds, glucoamylases cleaving 1,4 and 1,6-α-D glucosidic linkages, glycosyltransferases, or pullulanases cleaving 1,6-α-D glucosidic linkages, preferably one or more alpha amylases.

6. The method of any of the preceding claims, wherein the temperature of the suspension containing the at least one enzyme is raised on the drum drying device to a final temperature of not less than 120°C, preferably not less than 130°C, in particular 140 to 160°C.

7. The method of any of the preceding claims, wherein the final temperature of the suspension containing the at least one enzyme on the drum drying device is above the inactivation temperature of the at least one enzyme.

8. The method of any of the preceding claims, wherein the residence time of the suspension containing the at least one enzyme on the drum drying device is between 10 sec and 2 min.

9. The method of any of the preceding claims, wherein the starch obtained in step e) of claim 1 has a low viscosity of less than 50 mPa*s, preferably less than 30 mPa*s, more preferably less than 20 mPa*s.

10. The method of any of the preceding claims, wherein the starch obtained in step e) of claim 1 is a starch having a cold water solubility of 50 to 100%, in particular 100%.

11. The method of any of the preceding claims, wherein the starch suspension prepared according to step a) of claim 1 has a density of 20 to 26 Baume, in particular 22 to 24 Baumé.

12. Use of a drum drying device for the gelatinization of a suspension comprising native starch, water and at least one enzyme, wherein the suspension was not heated to a temperature above 70 °C, preferably not above 60 °C, in particular not above 50 °C before applying to the drum drying device.
